# EUROPÄISCHE PATENTSCHRIFT

(11) **EP 0 417 586 B1**
(45) Veröffentlichungstag und Bekanntmachung des Hinweises auf die Patenterteilung: **16.12.1998**
(21) Anmeldenummer: 90116845.0
(22) Anmeldetag: 03.09.1990
(51) Int. Cl.: C07F 7/18, C07F 7/12, B01J 20/32, C01B 33/159, C08G 77/26, C07H 23/00

(54) **Silanderivate**
Silane derivatives
Dérivés de silanes

(30) Priorität: 12.09.1989 DE 3930344
(43) Veröffentlichungstag der Anmeldung: 20.03.1991
(73) Patentinhaber: Kinkel, Joachim, Dr., 55452 Guldental (DE); Berndt, Heinz, Prof. Dr., 52146 Würselen (DE)
(72) Erfinder: Berndt, Heinz, Prof. Dr., D-5102 Würselen (DE); Höcker, Hartwig, Prof. Dr., D-5100 Aachen (DE); Kuropa, Rolf, D-5100 Aachen (DE); Kinkel, Joachim, Dr., D-6531 Guldental (DE)

(56) Entgegenhaltungen:
- WO-A-86/06072
- US-A- 2 973 383
- JOURNAL OF CHROMATOGRAPHY Bd. 481, 3. November 1989, AMSTERDAM Seiten 380 - 386; R. KUROPKA ET AL: 'Chiral stationary phases via hydrosilylation reaction of N-acryloylamino acids'

## Beschreibung

Die Erfindung betrifft Silanderivate der Formel I 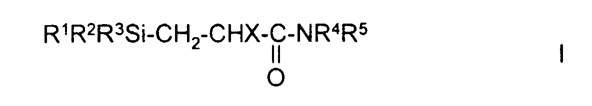 worin
- X: H oder CH₃,
- R¹, R² und R³: jeweils Halogen, Alkyl mit bis zu 12 C-Atomen, eine Phenyl-, Phenylalkyl-, Phenoxy-, Phenylalkoxygruppe, wobei die Phenylgruppe auch substituiert sein kann durch Halogen, Alkyl und/der Alkoxy, und worin jeweils die Alkylketten bis zu 12 C-Atome haben, oder einen Cycloalkyl- oder Alkylcycloalkylrest mit 3-10 C-Atomen im Ring , der durch Alkyl mit bis zu 12 C-Atomen substituiert sein kann, bedeutet, und
- R⁴ und R⁵: a) jeweils unabhängig voneinander H, eine Alkyl-, Naphthyl-, Naphthylalkyl-, Phenyl-, Phenylalkylgruppe, wobei diese Gruppen durch Alkyl, Cycloalkyl, Halogen, Cyano, Amino oder Hydroxy substituiert sein können, wobei die Alkylgruppen bis zu 12 C-Atome besitzen und worin gegebenenfalls ein oder zwei CH₂-Gruppen substituiert sein können durch

   -O-, -CO-, -CO-O-, -O-CO-, -CH=CH-, -NH-C(O)-, -C(O)-NH-,

   wobei nicht zwei Heteroatome miteinander verknüpft sind und R⁴ und R⁵ auch zusammen eine Alkylenbrücke mit bis zu 7 C-Atomen bilden können.
b) einen Cycloalkyl- oder Alkylcycloalkylrest mit 3-10 C-Atomen im Ring und bis zu 12 C-Atomen in der Alkylgruppe,
c) Mono- oder Oligosaccharide bedeutet, oder -NR⁴R⁵ ein über den Stickstoff gebundenes Aminosäurederivat mit bis zu 15 C-Atomen in der Hauptkette, oder R⁴ oder R⁵ eine oder mehrere zusätzliche (R¹R²R³Si(CH₂CHX)C(O)N)- Gruppen enthalten können,
mit der Maßgabe, daß mindestens einer der Reste R¹, R² und R³ eine reaktionsfähige Abgangsgruppe darstellt, sowie ein Verfahren zur Herstellung dieser Silane und deren Verwendung.

Reaktive Orgasilane werden zur Verbesserung und Veränderung von Oberflächen bei technischen Prozessen in der industrie und auch in der Forschung seit langem eingesetzt. Sie stellen wertvolle Hilfsmittel dar.

Mit reaktiven Silanverbindungen lassen sich beispielsweise die Benetzungseigenschaften von Pigmenten und Fülllstoffen beim Einarbeiten in Kunststoffe verbessern.

Auch die elektrischen Eigenschaften und die Beständigkeit gegen Wasser und Feuchtigkeit werden erhöht.

Silanderivate werden also zur Hydrophobisierung von Substraten eingesetzt.

Weiterhin wird die Dispergierbarkeit von Farben und Pigmenten durch den Einsatz von Silanen verbessert.

Meistens müssen die Silanderivate mit funtionellen Gruppen an den organischen Kunstdstoff oder Klebstoff, in welchen sie eingearbeitet werden sollen, angepaßt werden.

Dazu ist eine ganze Palette von Silanen mit den verschiedensten funktionellen Gruppen, wie z.B. Amino-, Mercapto-, Vinyl-, Epoxy-, Carboxyl- oder Methacrylgruppen notwendig.

Silane werden auch eingesetzt als Füllstoffe für Elastomere, als Haftvermittler oder Haftverstärker bei Phenol-, Furan-, Melamin- oder Epoxidharzen, oder auch als Zusatz für Bautenschutzmittel.

Die Verwendung von Silanen für die Hydrosilylierung von Alkenen und Alkinen in Gegenwart von Schwermetallsalzen bzw. alkalischen Katalysatoren ist ebenfalls seit langem bekannt und z.B. beschrieben von J.L. Speyer et al. in J. Am. Chem. Soc. 79 (1957) 974 - 979. Dieses Verfahren wird großtechnisch angewandt.

Ein weiteres Anwendungsgebiet für reaktive Silane ist das Gebiet der Chromatographiematerialien. Die Silane werden zur Oberflächenmodifizierung von chromatographischen Trägermaterialien eingesetzt. Die Eigenschaften von chromatographischen Trägermaterialien können so wunschgemäß verändert werden, z. B. Überführung eines hydrophilen Kieselgelmaterials in ein hydrophobes Material, oder auch in ein Material, das Amino-, Diol- oder Cyanogruppen enthält. Diese Oberflächenmodifizierung ist dem Fachmann aus zahlreichen Literaturstellen bekannt. Beispielsweise sind steuerbare Modifizierungen auch beschrieben in der DE-OS 34 27 923.

Um für jedes Trennproblem ein optimales Trennmaterial zu erhalten, benötigt man eine große Palette von reaktiven Silanderivaten. Beispielsweise gibt es noch keine geeigneten Silanisierungsmittel, um auf einfachem und leichten Wege Aminosäurederivate über das Stickstoffende auf einer Kieselgelmatrix zu binden. Solche Materialien sind hervoragend geeignet zur Auftrennung von optisch aktiven Substanzen.

Auch für die anderen Einsatzgebiete der Silane besteht ein Bedarf an neuen, reaktiven Silanen, um Produkte mit völlig andersartigen oder verbesserten Eigenschaften darzustellen.

Die Aufgabe der vorliegenden Erfindung bestand daher darin, neue, reaktive Silanderivate zu finden, die auf einfachem Wege herstellbar sind und für viele Anwendungen bei technischen Prozessen geeignet sind.

W. T. Black beschreibt zwar im US Patent 2,973,383 die Herstellung und Verwendung von Silanen, die unter anderem auch die Struktur R'R"NC(O)(CH₂)₂SiR¹R²R³ besitzen können, in diesen Fällen sind jedoch die reaktiven Abgangsgruppen R¹R²R³ immer alkoxyfunktional. Dasselbe gilt für die in der Anmeldeschrift WO 86/06072 genannten Silanverbindungen. Nach keinem der in diesen beiden Veröffentlichungen genannten Verfahren gelingt es, Silane mit Halogen(en) als nucleophiler, funktioneller Abgangsgruppe darzustellen, da sich in allen Fällen bevorzugt Silazane oder andere Kondensationsprodukte bilden würden, die den erfindungsgemäßen Verbindungen in keinster Weise ähneln.

Überraschenderweise wurde nun gefunden, daß durch die Hydrosilylierung von (Meth)acrylsäureamiden neue Silanderivate hergestellt werden können, die Halogene als Abgangsgruppen enthalten und sich allgemein hervorragend als Silanisierungsmittel eignen.

Gegenstand der Erfindung sind daher die Silanderivate der Formel I nach Anspruch 1. Gegenstand der Erfindung ist auch ein Verfahren zur Herstellung von Silanderivaten der Formel I, bei welchem (Meth)Acryl-amide der Formel II

CH₂ = CX-C(O)-NR⁴R⁵ II

mit Silanen der Formel III

R¹R²R³SiH III

in Gegenwart von Katalysatoren, die bekanntermaßen Hydrosilylierungsreaktionen zu katalysieren vermögen, umgesetzt werden, wobei X, R¹, R², R³, R⁴ und R⁵ die in Formel I angegebene Bedeutung haben. Die erfindungsgemäßen Silane können auch mehrwertig sein und weitere reaktive Reste der Formel IV

(R¹R²R³Si-CH₂-CHX-C(O)-N)= IV

enthalten, die sich aus entsprechend mehrfach mit (Meth)Acrylsäureamidgruppen substituierten Resten R⁴ und R⁵ in Formel II herleiten.

Ferner ist Gegenstand der Erfindung die Verwendung dieser Silanderivate zur Oberflächenbeschichtung von anorganischen Substraten, zur Oberflächenmodifizierung von chromatographischen Trägermaterialien sowie zur Herstellung von Polysiloxanen.

In den Formeln I, II und IV bedeutet X H oder CH₃, vorzugsweise H.

R¹, R² und oder R³ in den Formeln I, III und IV bedeuten vorzugsweise Halogen, wie z.b. Fluor, Chlor oder Brom, Alkyl mit bis zu 12 C-Atomen, vorzugsweise mit bis zu 7 C-Atomen. Die Alkylgruppen können geradkettig oder verzweigt sein und bedeuten demnach bevorzugt Methyl, Ethyl, Propyl, Butyl, Pentyl, Hexyl, Heptyl, Isopropyl, Isobutyl, sek.- oder tert.-Butyl, 3-Methylbutyl, 1.1-, 1.2- oder 2.2-Dimethylpropyl, 1-ethylpropyl, ferner auch Octyl, Nonyl, Decyl, Undecyl und Dodecyl.

Ferner bedeuten R¹, R² und oder R³ auch bevorzugt eine Phenyl-, Phenylalkyl-, Phenoxy- oder Phenylalkoxygruppe wobei die Phenylgruppe ein- oder mehrfach, vorzugsweise ein- oder zweifach, insbesondere bevorzugt einfach substituiert sein kann durch Halogen, wie Fluor, Chlor oder Brom, wobei Fluor und Chlor bevorzugt sind, Alkyl und oder Alkoxy. Für die in diesen Gruppen enthaltenen Alkyl- bzw. Alkoxygruppen gelten die oben genannten bevorzugten Bedeutungen. Beispielhaft werden folgende Gruppen als Vertreter genannt: Phenyl, Phenoxy, Benzyl, 1- oder 2-Phenethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenyl-ethyl, o-, m- oder p-Methoxyphenyl, 1- oder 2-o-, Methoxyphenylethyl, o-, m- oder p-Fluorphenyl, o-, -m- oder p-Chlorphenyl, o-, m- oder p-Chlorbenzyl, 1- oder 2-o-, -m- oder -p-Chlorphenylethyl, o-, m- oder p-Bromphenyl, o-, m- oder p-lodphenyl, 2.3-, 2.4-, 2.5-, 2.6-, oder 3.5-Dimethoxyphenyl, 2.3-, 2.4-, 2.5-, 2.6-, 3.4- oder 3.5-Dimethoxybenzyl.

Weiterhin bevorzugt sind für R¹, R² und oder R³ die Bedeutungen Cycloalkyl oder Alkylcycloalkylrest mit 3-10 C-Atomen im Ring, der auch durch Alkyl mit bis zu 12 C-Atomen substituiert sein kann. Der Cycloalkylrest bedeutet demnach bevorzugt Cyclopropyl, Cyclobutyl, Cyclopentyl, Cyclohexyl, Cycloheptyl, Cyclooctyl, Cyclononyl oder Cyclodecyl. Für die Alkylgruppen kommen die bereits gegebenen bevorzugten Bedeutungen in Frage. Beispielhafte Vertreter sind: 4-Ethylcyclohexyl, 4-Propylcyclohexyl, 4-Butylcyclohexyl, 4-Isopropylcyclohexyl oder auch 3-Ethylcyclopropyl oder 5-Ethylcylooctyl. Cyclohexylmethyl, 1- oder 2-Cyclohexylethyl, 2-, 3- oder 4-methylcyclohexylmethyl, 2-, 3- oder 4-ethylcyclohexylmethyl, 2-, 3- oder 4-tert.-Butylcyclohexylmethyl, 1- oder 2-(2-, (3- oder (4-Ethylcyclohexyl)-ethyl.

Für die Reste R¹, R² und oder R³ besteht die Maßgabe, daß mindestens einer dieser Reste eine reaktionsfähige Gruppe darstellt.

Diese reaktionsfähige Gruppe stellt vorzugsweise eine nukleophile Abgangsgruppe dar, und bedeutet demnach vorzugsweise -F, -Cl, Br oder OPhenyl. Insbesondere ist -Cl bevorzugt.

Folgende Verbindungen der Formel III sind besonders bevorzugt:

Dimethylchlorsilan, Dichlorsilan, Phenyldichlorsilan, Methyldichlorsilan, Diethylchlorsilan, Ethyldichlorsilan, Trichlorsilan,

R⁴ und R⁵ in den Formeln I und II bedeuten jeweils unabhängig voneinander vorzugsweise H oder eine Alkylgruppe, wobei für die Alkylgruppen vorzugsweise die bereits für R¹, R² und R³ genannten bevorzugten Bedeutungen in Frage kommen.

Zusätzlich sind für R⁴ und oder R⁵ auch Alkylgruppen bevorzugt, worin eine oder zwei CH₂-Gruppen durch

-CH=CH- und/der -NHC(O)- oder -C(O)NH-

ersetzt sind.
R⁴ und oder R⁵ bedeuten auch vorzugsweise eine Phenyl- oder Phenylalkylgruppe. Die Phenylgruppe kann substituiert sein durch Halogen, vorzugsweise durch F, Cl, Br, durch Amino, Cyano, Hydroxy oder durch Alkyl. Für Alkyl stehen auch hier wieder vorzugsweise die bereits angegebenen bevorzugten Bedingungen.

Ferner ist auch eine Naphthylgruppe für R⁴ und R⁵ bevorzugt, die ebenfalls durch die bei der Phenylgruppe genannten Substituenten substituiert sein kann. R⁴ und R⁵ bedeuten ferner auch auch einen Cycloalkyl- oder Alkylcycloalkylrest. Die bevorzugren Bedeutungen für diese Gruppen entsprechen den berweits für R¹, R² und R³ angegebenen Bedingungen.
Der Cycloalkylrest kann auch als Substituent an einer Alkylgruppe stehen und beispielsweise 1-Cyclohexylethyl, Cyclopropyl oder 2-Cyclohexylbutyl bedeuten.

R⁴ und R⁵ können auch zusammen eine Alkylengruppe mit bis zu 7 C-Atomen, vorzugsweise 3-7 C-Atomen bilden und bedeuten demnach vorzugsweise auch Propylen. Butylen. Pentylen. Hexylen oder Heptylen.

R⁴ und oder R⁵ bedeuten vorzugsweise auch einen Mono- oder Oligosaccaridrest. Bevorzugt sind Mono- oder Disaccaride, wie z. b. Glucosyl-, Arabinosyl-, Ribosyl-, Galactosyl-, Fructosil-, Lactose- oder Saccarose-Reste. Die Saccaridreste können cyclisch oder offenkettig vorliegen.

Als Reste kommen allgemein Pentosen, Hexosen, Fructosen, Di-oder Trisaccaride, auch Glycoside, Pyranosen, Furanosen, Pyranoside oder Furanoside in Frage.

Besonders bevorzugt sind solche Verbindungen der Formeln I und II, worin einer der Reste R⁴ und R⁵ H bedeutet.

In den Formeln I und II bedeutet -NR⁴R⁵ vorzugsweise auch ein über den Stickstoff gebundenes Aminosäurederivat mit bis zu 15 C-Atomen in der Hauptkette. Als Aminosäurederivat können Ester, Amide, die freien Säuren oder auch am N substituierte Amide eingesetzt werden.

Bevorzugt sind Aminosäurealkylester mit bis zu 7 C-Atomen in der Alkylkette, wobei diese geradkettig oder verzweigt sein kann. Ferner bevorzugt sind Phenylester. Der Substituent an der evtl. vorhandenen Amidgruppe ist vorzugsweise eine Alkylgruppe mit bis zu 7 C-Atomen, die ebenfalls geradkettig oder verzweigt sein kann, oder eine Phenylgruppe.

Alle allgemein bekannten und gängigen Aminosäuren können hier eingesetzt werden. Vorzugsweise ist-NR⁴R⁵ ein Derivat, das sich von folgenden Aminosäuren ableitet: Glycin, ß-Alanin, Alanin, £-Aminocapronsäure, Phenylalanin, a-Aminoisobuttersäure, 4-Aminobenzoesäure, Valin, Leucin, Isoleucin, Serin, Threonin, Asparaginsäure, Asparagin, Glutaminsäure, Glutamin, Lysin, Arginin, Histidin, Tyrosin, Tryptophan, oder Ornitin. Vor allem sind die Methyl-, Ethyl-, oder Propylester dieser Aminosäuren bevorzugt.

Im Folgenden ist eine kleinere Gruppe von besonders bevorzugten Verbindungen der Formel II angegeben:

CH₂ =CX-CO-NH-CH₂-CH₃

CH₂ =CX-CO-NH-C(CH₃)₃

CH₂ =CX-CO-NH-CH(CH₃)-Phenyl

CH₂ =C(CH₃)-CO-NH-CH₂-CH₂NH₂

CH₂ =CX-CO-N(C₂H₅)-CH₂-CH₂OH

CH₂ =CX-CO-NH-CH(CH₃)-C₆H₁₁

CH₂ =CX-CO-NH-CH₂-CH₂CH₂-NH-CO-CX=CH₂

CH₂ =CX-CO-NH-CH₂-CH₂-Phenyl

CH₂ =CX-CO-NH-Phenyl

CH₂ =CX-CO-NH-p-Alkylphenyl

CH₂ =CX-CO-NH-Alkyl

CH₂ =CX-CO-NH-C₆H₁₁

CH₂ =CX-CO-NH-CH(Alkyl)(C₆H₁₁)

CH₂ =CX-CO-NH-Alkyl

CH₂ =CX-CO-NH-CH(Phenyl)(COOC₂H₅)

CH₂ =CX-CO-NH-CH₂(COOC₂H₅)

CH₂ =CX-CO-NH-CH(CH₃)(COOAlkyl)

CH₂ =CX-CO-NH-CH(COOAlkyl)(CH(CH₃)₃

CH₂ =CX-CO-NH-CH(COOAlkyl)Phenyl

CH₂ =CX-CO-NH-2-Naphthyl

CH₂ =CX-CO-NH-CH₂-CH₂-(p-Aminophenyl)

CH₂ =CX-CO-NH-(p-Chlormethylphenyl)

CH₂ =CX-CO-N(C₃H₇)(C₄H₉)

CH₂ =CX-CO-N(C₄H₈)

CH₂ =CX-CO-N(C₅H₁₀)

CH₂ =CX-CO-NH(CH₂)₃CN

CH₂ =CX-CO-NH-CH₂-(p-Alkyloxyphenyl)

CH₂ =CX-CO-NH-(tetra-O-acetylglucosyl)

CH₂ =CX-CO-NH-1-(2-Methyl-5-lsopropylcyclohexan)

Die Verbindungen der Formel II können allgemein hergestellt werden durch Umsetzen der entsprechenden Amine oder Aminosäurederivate R⁴R⁵NH mit reaktionsfähigen Methacrylsäure- oder Acrylsäurederivaten, vorzugsweise in Gegenwart eines Polymerisationsinhibitors bei Temperaturen zwischen etwa -5 °C und 60 °C. Vorteilhafterweise arbeitet man bei Raumtemperatur unter Verwendung eines inerten organischen Lösungsmittels, insbesondere eines Halogenkohlenwasserstoffs wie Methylenchlorid oder Chloroform. Die Reaktionszeiten liegen zwischen etwa 30 Minuten und 4 Stunden und hängen im Wesentlichen von der Reaktionstemperatur ab. Die Reaktionsbedingungen sind in der Literatur vielfältig beschrieben.

Gegenstand der Erfindung ist auch das Verfahren zur Herstellung der Silanderivate der Formel I. Dieses Verfahren entspricht der an sich bekannten Hydrosilylierung von Alken- oder Alkinverbindungen, jedoch ist dieses Verfahren bisher noch nie auf (Meth)Acrylamide als Ausgangsverbindungen angewandt worden.

Überraschenderweise wurde nun gefunden, daß sich (Meth)Acrylamide der Formel II mit Silanen der Formel III in Gegenwart von Katalysatoren, die bekanntermaßen Hydrosilylierungsreaktionen zu katalysieren vermögen, hydrosilylieren lassen.

Die allgemein bekannten Reaktionsbedingungen können aus der Literatur entnommen werden, beispielsweise J. Am. Chem. Soc. 79 (1957) 974 - 979.

Üblicherweise arbeitet man in inerten organischen Lösungsmitteln, insbesondere in Halogenkohlenwasserstoffen, Alkoholen, Ethern, Kohlenwasserstoffen oder aromatischen Kohlenwasserstoffen.

Vorzugsweise erfolgt die Umsetzung bei Temperaturen zwischen 0° und 140 °C, insbesondere bevorzugt bei Temperaturen zwischen 20° und 100 °C. Als Katalysatoren werden die für die Hydrosilylierungsreaktion aus der Literatur bekannten Schwermetallsalze bzw. alkalische Katalysatoren beigesetzt. Als bevorzugte Katalysatoren kommen z.B. Schwermetallsalze wie K₂PtCl₄, Rutheniumsalze wie RuCl₃, Palladiumsalze, Platin/Kohle oder auch lridiumsalze in Frage. Insbesondere bevorzugt wird Hexachloroplatinsäure eingesetzt.

Die Reaktionszeiten liegen zweckmäßiger Weise zwischen 30 Minuten und 30 Stunden, vorzugsweise zwischen 1 und 10 Stunden. Die Aufarbeitung und Reinigung erfolgt zweckmäßig durch Destillation oder Umkristallisation.

Wenn im Ausgangsmolekül der Formel II zwei oder auch mehrere CH₂ = CX-C(O)-N= -Gruppen enthalten sind, wie z.B. in Ethylenbisacrylamid, enthält man bei Zugabe der entsprechenden höheren Menge an Silanen der Formel III das an allen CH₂ = CX-C(O)-N=-Gruppen hydrosilylierte Produkt.

Mit diesem Verfahren, das erfindungsgemäß erstmalig auf (Meth)Acrylamide als Ausgangsprodukte angewandt wird, steht somit eine einfache Darstellungsmethode für eine Vielzahl von neuen Silanderivaten zur Verfügung, die die besonders reaktiven Halogenabgangsgruppen im Molekül aufweisen.

Die erfindungsgemäßen Silanderivate können generell für alle bekannten Anwendungen von reaktiven Silanen verwendet werden, die bereits eingangs beschrieben wurden.

Vor allem sind die erfindungsgemäßen Silanderivate zur Oberflächenmodifizierung geeeignet, insbesondere auch zur Oberflächenmodifizierung von chomatographischen Trägermaterialien und zur Oberflächenbeschichtung anorganischer Materialien wie z.B. anorganischen Oxiden.

Mit den erfindungsgemäßen Verbindungen ist es beispielsweise auch möglich, mit Aminosäurederivaten oberflächenmodifizierte Trägermaterialien zu erhalten, bei welchen das Aminosäurederivat über das StickstoffEnde an den Träger gekuppelt ist. Diese Bindungsart hat gegenüber der sehr viel mehr verbreiteten Kupplung über das C-Ende den Vorteil, daß Standardmethoden der Peptidchemie angewandt werden können, um beispielsweise weitere Gruppen an das C-Ende zu kuppeln. Diese Bindung über das N-Ende ist bisher nur über aufwendige Umwege möglich gewesen.

Als chromatogrsaphische Träger kommen habei alle im Handel befindlichen Materialien in Frage. Insbesondere bevorzugt sind Träger auf Basis von Kieselgel, TiO₂ oder Al₂O₃.

Die Oberflächenmodifizierung mit den erfindungsgemäßen Silanen erfolgt nach literaturbekannten Methoden.

Die Silanderivate der Formel I und IV können auch optisch aktiv sein. Die daraus resultierenden modifizierten Trägermaterialien sind dann hervorragend zur Trennung von racemischen Gemischen in ihre optischen Antipoden geeignet.

Die Silanderivate der Formel I können ferner zur Herstellng von Polysiloxanen verwendet werden. Dafür können allgemein bekannte Reaktionsbedingungen aus den entsprechenden Fachbüchern gewählt werden.

Die folgenden Herstellungs- und Anwendungsbeispiele sollen die erfindungsgemäßen Silanderivate, deren Herstellung und Verwendung näher erläutern.

### Beispiel 1

3.20 g (0.13 mol) Acryloyl-D-phenylglycyl-n-propylamid [Darstellung: Benzyloxycarbonylphenylglycin wird nach der gemischten Anhydridmethode mit n-Propylamin umgesetzt, dann wird die Schutzgruppe durch katalytische Hydrierung abgespalten, anschließend wird mit Acrylsäurechlorid umgesetzt zum entsprechenden Acrylamidderivat]. 4.50 g (0.48 mol) Dimethylchlorsilan und 100 mg Hexachloroplatinsäure werden unter stickstoffatmosphäre in 120 ml Chloroform suspendiert und 2 Stunden am Rückfluß erhitzt. Nach Eindampfen zur Trockne erhält man das entsprechende Dimethylchlorsilylderivat: N-(3-Dimethyl- chlorsilylpropionyl)-D-phenylglycyl-n-propylamid.

### Beispiel 2

Zu einem Gemisch aus N-(3-Dimethylchlorsilylpropionyl)-D-phenylglycyl-n-propylamid (Darstellung siehe Beispiel 1) und 50 ml Pyridin gibt man 2,4 g Silicagel 824 Stunden getrocknet bei 160 °C/0.05 mbar) und rührt bei 40 °C 24 Stunden lang. Das so modifizierte Kieselgel kann bei Bedarf noch zusätzlich mit Trimethylchlorsilan umgesetzt werden (sog. endcapping).

Das Gel wird gewaschen und getrocknet. Es enthält 0.54 mmol chirale Gruppen und ist hervorragend zur chromatographischen Trennung von Enantiomeren geeignet.

### Beispiel 3

Das in Beispiel 2 hergestellte modifizierte Kieselgel wird nach bekannter Art und Weise in eine Chromatographiesäule gepackt.

Folgende Trennungen racemischer Aminosäurederivate wurden durchgeführt:
Trennbedingungen:
Säule 200 x 4.0 mm I.D.
HPLC-Gerät: Perkin-Elmer Series 2B
Detektor: Perkin-elmer LC 55
Flußrate: 1 ml/min
Probenlösung: 0,2 - 0,5 mg/ml (in Ethylacetat)
Aufgabe durch eine 10 µl-Schleife

In Tabelle 1 sind die Trennfaktoren (α) der verschiedenen N^{α} -3,5-Dinitrobenzoylaminosäure-2-propylester, die nach den genannten Bedingungen aufgetrennt wurden, aufgelistet.

Aus der Tabelle ist klar zu erkennen, daß eine hervorragende Trennung der Enantiomere erfolgt.

**Tab. 1**

| Enantiomerentrennung von N^{α}-3,5-Dinitrobenzoylaminosäure-2-propylestern | | | | |
|---|---|---|---|---|
| k_{D} = Kapazitätsfaktor für das D-Isomer, | | | | |
| k_{L} = Kapazitätsfaktor für das L-Isomer | | | | |
| MP = Mobile Phase (%,v/v 2-Propanol in Hexan) | | | | |

| Aminosäure | k_{D} | k_{L} | α | MP |
|---|---|---|---|---|
| Alanin | 1.62 | 2.68 | 1.65 | 10 |
| Valin | 0.75 | 1.97 | 2.63 | 10 |
| Leucin | 0.90 | 1.95 | 2.17 | 10 |
| lsoleucin | 0.86 | 2.24 | 2.62 | 10 |
| Phenylalanin | 1.26 | 2.76 | 2.19 | 10 |
| Phenylglycin | 1.09 | 2.00 | 1.84 | 10 |
| Tyrosin | 0.66 | 1.46 | 2.21 | 30 |
| Serin | 3.03 | 3.60 | 1.19 | 10 |
| Threonin | 2.14 | 2.50 | 1.17 | 10 |
| Lysin | 9.15 | 12.37 | 1.35 | 10 |
| Glutaminsäure | 0.81 | 1.29 | 1.60 | 10 |

### Beispiel 4

Analog Beispiel 3 erhält man sehr gute Trennungen von racemischen Gemischen von verschiedenen N^{α}-3,5-Dinitrobenzoylaminosäure-2-propylamiden.

In Tabelle 2 sind die Resultate zusammengefaßt.

**Tab. 2**

| Enantiomerentrennung von N^{α}-3,5-Dinitrobenzoylaminosäure-2-propylamiden | | | | |
|---|---|---|---|---|
| k_{D} = Kapazitätsfaktor für das D-Isomer, | | | | |
| k_{L} = Kapazitätsfaktor für das L-Isomer | | | | |
| MP = Mobile Phase (%,v/v 2-Propanol in Hexan) | | | | |

| Aminosäure | k_{D} | k_{L} | α | MP |
|---|---|---|---|---|
| Alanin | 2.29 | 12.18 | 5.32 | 10 |
| Valin | 0.56 | 5.76 | 6.01 | 10 |
| Leucin | 0.88 | 5.80 | 6.58 | 10 |
| lsoleucin | 0.84 | 5.35 | 6.35 | 10 |
| Phenylalanin | 1.95 | 13.22 | 6.77 | 10 |
| Phenylglycin | 1.84 | 6.59 | 3.59 | 10 |
| Serin | 6.67 | 18.95 | 2.84 | 10 |
| Threonin | 3.25 | 10.95 | 3.37 | 10 |
| Prolin | 3.08 | 2.71 | 1.14 | 10 |
| Lysin | 13.70 | 46.66 | 3.40 | 10 |
| Tryptophan | 4.53 | 34.46 | 7.60 | 10 |
| Glutaminsäure | 3.53 | 6.53 | 1.85 | 10 |

In Tabelle 3 sind die Ergebnisse der zu Tabelle 2 analogen Trennungen aufgeführt. Lediglich die Zusamensetzung der mobilen Phase wurde geändert.

**Tab. 3**

| Enantiomerentrennung von N^{α}-3,5-Dinitrobenzoylaminosäure-2-propylamiden | | | | |
|---|---|---|---|---|
| k_{D} = Kapazitätsfaktor für das D-Isomer, | | | | |
| k_{L} = Kapazitätsfaktor für das L-Isomer | | | | |
| MP = Mobile Phase (%,v/v 2-Propanol in Hexan) | | | | |

| Aminosäure | k_{D} | kL | α | MP |
|---|---|---|---|---|
| Alanin | 0.51 | 2.84 | 5.60 | 30 |
| Valin | 0.27 | 1.71 | 6.30 | 30 |
| Leucin | 0.25 | 1.69 | 6.76 | 30 |
| lsoleucin . | 0.22 | 1.61 | 7.30 | 30 |
| Phenylalanin | 0.54 | 3.54 | 6.60 | 30 |
| Phenylglycin | 0.47 | 1.56 | 3.34 | 30 |
| Tyrosin | 0.88 | 6.77 | 7.70 | 30 |
| Lysin | 1.50 | 4.49 | 2.99 | 30 |
| Tryptophan | 0.63 | 4.56 | 7.27 | 30 |
| Glutaminsäure | 0.51 | 0.95 | 1.85 | 30 |
| Serin | 1.17 | 3.04 | 2.61 | 30 |
| Threonin | 0.70 | 2.22 | 3.16 | 30 |

Analog Beispiel 3 werden Trennungen racemischer Gemische von N^{α}-3,5-Dinitrobenzoylaminosäuredimethyl- bzw. -diethylamiden durchgeführt. Die entsprechenden Resultate sind in den Tabellen 4 und 5 zusammengefaßt.

**Tab. 4**

| Enantiomerentrennung von N^{α}-3,5-Dinitrobenzoylaminosäuredimethylamiden | | | | |
|---|---|---|---|---|
| k_{D} = Kapazitätsfaktor für das D-lsomer, | | | | |
| k_{L} = Kapazitätsfaktor für das L-lsomer | | | | |
| MP = Mobile Phase (%,v/v 2-Propanol in Hexan) | | | | |

| Aminosäure | k_{D} | kL | α | MP |
|---|---|---|---|---|
| Alanin | 0.91 | 4.13 | 4.52 | 30 |
| Valin | 0.34 | 2.36 | 6.91 | 30 |
| Leucin | 0.29 | 2.41 | 8.36 | 30 |
| lsoleucin | 0.27 | 2.24 | 8.19 | 30 |
| | | | | |

| Abb. 5 | | | | |
|---|---|---|---|---|
| Enantiomerentrennung von N^{zα}-3,5-Dinitrobenzoylaminosäurediethylamiden | | | | |
| k_{D} = Kapazitätsfaktor für das D-lsomer, | | | | |
| k_{L} = Kapazitätsfaktor für das L-lsomer | | | | |
| MP = Mobile Phase (%,v/v 2-Propanol in Hexan) | | | | |

| Aminosäure | kD | kL | α | MP |
|---|---|---|---|---|
| Alanin | 0.44 | 1.85 | 4.21 | 30 |
| Valin | 0.18 | 1.15 | 6.34 | 30 |
| Isoleucin | 0.18 | 1.24 | 6.72 | 30 |

## Patentansprüche

1. Silanderivate der Formel I, 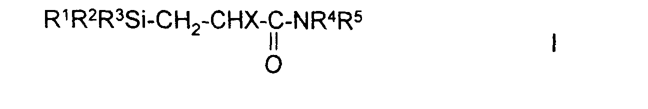 worin
X H oder CH₃,
R¹,R² und R³ jeweils Halogen, Alkyl mit bis zu 12 C-Atomen, eine Phenyl-, Phenylalkyl-, Phenoxy-, Phenylalkoxygruppe, wobei die Phenylgruppe auch substituiert sein kann durch Halogen, Alkyl und/oder Alkoxy, und worin jeweils die Alkylketten bis zu 12 C-Atome haben, oder einen Cycloalkyl- oder Alkylcycloalkylrest mit 3-10 C-Atomen im Ring , der durch Alkyl mit bis zu 12 C-Atomen substituiert sein kann,
bedeutet, und
R⁴ und R⁵
a) jeweils unabhängig voneinander H, eine Alkyl-, Naphthyl-, Naphthylalkyl-, Phenyl-, Phenylalkylgruppe, wobei diese Gruppen durch Alkyl, Cycloalkyl, Halogen, Cyano, Amino oder Hydroxy substituiert sein können, wobei die Alkylgruppen bis zu 12 C-Atome besitzen und worin gegebenenfalls ein oder zwei CH₂-Gruppen substituiert sein können durch
-O-, -CO-, -CO-O-, -O-CO-, -CH=CH-, -NH-C(O)-,-C(O)-NH-,
wobei nicht zwei Heteroatome miteinander verknüpft sind, R₄ und R₅ auch zusammen eine Alkylenbrücke mit bis zu 7 C-Atomen bilden können,
b) einen Cycloalkyl- oder Alkylcycloalkylrest mit 3-10 C-Atomen im Ring und bis zu 12 C-Atomen in der Alkylgruppe,
c) Mono- oder Oligosaccharide bedeutet,
oder
-NR⁴R⁵ ein ein über den Stickstoff gebundenes Aminosäurederivat mit bis zu 15 C-Atomen in der Hauptkette,
oder
R⁴ oder R⁵ eine oder mehrere zusätzliche R¹R²R³Si(CH₂CHX)C(O)N-gruppe enthalten, wobei R¹, R², R³ sowie X weiterhin die in Formel I angegebene Bedeutung haben,
mit der Maßgabe, daß mindestens einer der Reste R¹, R² und R³ eine reaktionsfähige Abgangsgruppe ausgewählt aus Cl, Br oder F darstellt.

2. Silane nach Anspruch 1, dadurch gekennzeichnet, daß sie optisch aktiv sind.

3. Verfahren zur Herstellung von Silanderivaten in Anspruch 1 und 2, dadurch gekennzeichnet, daß (Meth)Acrylamide der Formel II 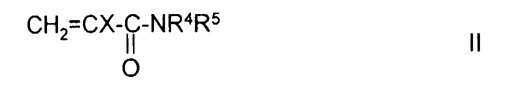 mit Silanen der Formel III
R¹R²R³SiH III
in Gegenwart von Katalysatoren, die bekanntermaßen Hydrosilylierungsreaktionen zu katalysieren vermögen, umgesetzt werden, wobei X, R¹, R², R³, R⁴ und R⁵ die in Anspruch 1 angegebene Bedeutung haben.

4. Verwendung der Silanderivate nach Anspruch 1 und 2 zur Oberflächenmodifizierung von chromatographischen Trägermaterialien.

5. Verwendung der Silanderivate gemäß Anspruch 1 und 2 zur Herstellung von Polysiloxanen.

## Claims

1. Silane derivates according to formula I, 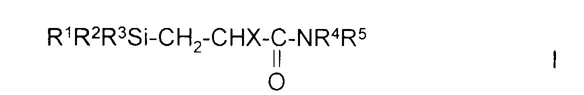 wherein
X is H oder CH₃,
R¹, R² and R³ are in each case, independently of one another, halogen, alkyl having up to 12 C atoms, a phenyl-, phenylalkyl-, phenoxy-, phenyl-alkoxy group, wherein the phenyl group can also be substituted by halogen, alkyl and/or alkoxy, and wherein the alkyl chains in each case have up to 12 C atoms, or a cycloalkyl or alkylcycloalkyl radical having 3-10 C atoms in the ring, which can be substituted by alkyl having up to 12 C atoms, and
R⁴ und R⁵
a) in each case independently of one another are H, an alkyl, naphthyl,
naphthylalkyl, phenyl, phenylalkyl group, it being possible for these groups to be substituted by alkyl, cycloalkyl, halogen, cyano, amino or hydroxyl, wherein the alkyl groups have up to 12 C atoms and wherein one or two CH₂ groups can optionally be substituted by
-O-, -CO-, -CO-O-, -O-CO-, -CH=CH-, -NH-C(O)-, -C(O)-NH-,
but wherein two hetero atoms are not linked to one another and R⁴ and R⁵ together can also form an alkylene bridge having up to 7 C atoms,
b) are a cycloalkyl or alkylcycloalkyl radical having 3 to 10 C atoms in the ring and up to 12 C atoms in the alkylgroup, or
c) mono or oligosaccharides, or -NR⁴R⁵ is an amino acid derivative which is bonded via the nitrogen and has up to 15 C atoms in the main chain, or R⁴ oder R⁵ contain one or multiple additional
(R¹R²R³Si(CH₂CHX)C(O)N)
groups, wherin R¹, R², R³ and X have the meaning given by formula I, with the proviso that at least one of the radicals R¹, R² and R³ is a reactive leaving group of Cl, Br or F.

2. Silanes according to claim 1, being optically active.

3. Process for the preparation of silane derivatives according to claim 1 and 2, in which (meth)acrylamides of the formula II 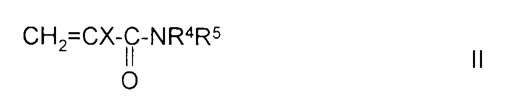 are reacted with silanes of the formula III
R¹R²R³SiH III
in the presence of catalysts which are known to be capable of catalyzing hydrosilylation reactions, X, R¹, R², R³, R⁴ and R⁵, having the meaning given in claim 1.

4. Use of the silane derivatives according claim 1 and 2 for the surface modification of chromatographic carrier materials.

5. Use of the silane derivatives according claim 1 and 2 for the preparation of polysiloxanes.

## Revendications

1. Les derivés de silane décrits par la formule 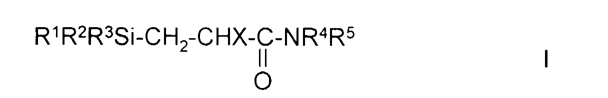 pour lesquels
X corresponds à H ou CH₃,
R¹, R² et R³ sont, dans chacun des cas et indépendament l'un d'autre, un halogène, un groupement alkyle ayant jusqu'à 12 atomes C, un groupement phényle, phénylalkyle, phénoxy, phényl, alkoxy pour lesquels le groupe phényle peut également être substitué par un halogène, un groupement alkyle et/ou alkoxy, et pour lesquels la chaîne alkyle possède, dans chacun des cas, jusqu'à 12 atomes C, ou un radical cycloalkyle ou alkylcycloalkyle ayant un cycle de 3 à 10 atomes C, qui peut être substitué par une chaîne alkyle possédant jusqu'à 12 atomes C, et pour lesquels
R⁴ et R⁵
a) sont, dans chacun des cas, indépendament l'un de l'autre, un atome H, un groupement alkyle, naphthyle, naphthylalkyle, phényle, phénylalkyle, chacun de ces groupes pouvant être substitués par alkyle, cycloalkyle, halogène, cyano, amino ou hydroxyle, pour lesquels les groupes alkyle ont jusqu'à 12 atomes C et pour lesquels un ou deux groupe CH2 peut optionnellement être substitué par
-O-, -CO-, -CO-O-, -O-CO-, -CH=CH-, -NH-C(O)-, -C(O)-NH-,
mais pour lesquels deux hetero atomes ne sont par liés l'un à l'autre et R⁴ et R⁵ peuvent également former ensemble un pont alkylène ayant jusqu'à jusqu'à 7 atomes C,
b) sont un radical cycloalkyle ou alkylcycloalkyle ayant un cycle de 3 à 10 atomes C, une chaîne alkyle possédant jusqu'à 12 atomes C, ou
des mono- ou oligosaccharides, ou
-NR⁴R⁵ est un dérivé d'acide aminé, greffé par l'atome d'azote et qui possède jusqu'à 15 atomes C sur la chaine principale, ou
R⁴ ou R⁵ contient un ou plusieurs groupements additionnels
(R¹R²R³Si(CH₂CHX)C(O)N)
pour lesquels R¹ ,R², R³ et X ont la signification décrite pour la formule I, dans la mesure où au moins un des radicaux R¹ ,R², R³ est un groupe réactif partant Cl, Br ou F.

2. Les silanes décrits dans la revendication 1 et qui sont optiquement actifs.

3. Le procédé de préparation des dérivés de silane décrits dans la revendication 1 et 2, pour lesquels des amides (meth)acryliques de formule II
CH₂ = CX-C(O)-NR⁴R⁵ II
réagissent avec des silanes de formule III
R¹R²R³SiH III
en présence de catalysateurs connus pour être capable de catalyser des réactions d'hydrosilysation, X, R¹, R², R³, R⁴ et R⁵, ayant le sens donné dans le cas de la formule I.

4. L'utilisation des dérivés de silane décrits dans la revendication 1 et 2 utilisés pour la modification de la surface des supports chromatographiques

5. L'utilisation des dérivés de silane décrits dans la revendication 1 et 2 utilisés pour la préparation des polysiloxanes.
